(19) Europäisches Patentamt
European Patent Office
Office européen des brevets

(11) Publication number: **0 081 384 B1**

(12) **EUROPEAN PATENT SPECIFICATION**

(45) Date of publication of patent specification: **02.04.86**

(21) Application number: **82306536.2**

(22) Date of filing: **08.12.82**

(51) Int. Cl.[4]: **C 07 C 51/10,** C 07 C 63/36, C 07 C 63/06, C 07 C 63/04, C 07 C 65/21, C 07 C 63/26, C 07 C 63/70, C 07 C 63/44, C 07 D 333/38, C 07 D 213/79, C 07 D 307/68

(54) **Process for the preparation of aromatic or heteroaromatic carboxylated compounds.**

(30) Priority: **09.12.81 IT 2550281**

(43) Date of publication of application: **15.06.83 Bulletin 83/24**

(45) Publication of the grant of the patent: **02.04.86 Bulletin 86/14**

(84) Designated Contracting States: **BE DE FR GB NL**

(56) References cited:
**GB-A-2 026 478**
**GB-A-2 079 748**

**CHEMICAL ABSTRACTS, vol. 87, no. 17, 24th October 1977, page 659, no. 134325q, Columbus Ohio (USA); H. ALPER et al.: "Phase-transfer catalyzed organometallic chemistry. The cobalt carbonyl-catalyzed carbonylation of halides"**

**CHEMICAL ABSTRACTS, vol. 87, no. 17, 24th October 1977, page 659, no. 134326r, Columbus Ohio (USA); L. CASSAR et al.: "Phase-transfer catalysis in cobalt-catalyzed carbonylation of benzyl halides"**

(73) Proprietor: **Montedison S.p.A.**
**31, Foro Buonaparte**
**I-20121 Milan (IT)**

(72) Inventor: **Foa', Marco**
**19, via del Sabbione**
**Novara (IT)**
Inventor: **Bencini, Elena**
**170, C.so XXIII Marzo**
**Novara (IT)**

(74) Representative: **Whalley, Kevin et al**
**Marks & Clerk 57/60 Lincoln's Inn Fields**
**London WC2A 3LS (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of aromatic or hetero-aromatic carboxylated compounds.

More particularly, the present invention relates to a process for the synthesis of aromatic or hetero-aromatic carboxylated compounds in the form of acids, esters and alkaline salts.

Specifically, this invention aims to provide a catalytic process for the synthesis of aromatic or hetero-aromatic carboxylated compounds, conducted in the presence of a cobalt based catalyst.

The (hetero)-aromatic mono- or bi-carboxylated compounds, obtained according to the present invention, have the following general formula (I):

$$Y{-}Ar{-}\underset{\underset{O}{\|}}{C}{-}OR \qquad (I)$$

wherein

Ar represents an aromatic nucleus having one or more benzoic rings optionally (poly)-condensed, or a hetero-aromatic nucleus optionally condensed, and wherein

Y represents a hydrogen atom or a halogen selected from Cl, Br and I, an alkyl, alkoxyl, or trifluoroalkyl group having up to 4 carbon atoms, an ester group, an aryloxy group, a nitrile group, an amide ($-CONH_2$) group, an acetamide ($CH_3CONH-$) group, an amine group, or an acyl ($R''-CO-$) group wherein R″ represents an alkyl or aryl group, or Y represents a $-CH_2COOR$ group wherein R represents a hydrogen atom, an alkyl group having up to 4 carbon atoms, or wherein R represents an alkali or an alkaline-earth metal, preferably selected from Na, K, Li and Ca.

The carboxylated compounds of formula (I) are interesting products that find useful application over a wide range of commercial appliances.

In fact, they may be used, for instance, in the preparation of alkydic resins (as for benzoic acid), dye-stuffs and pigments (as for naphthoic acid), and azo-dye-stuffs (anthranilic acid), as well as in the field of plasticizers (esters of higher alcohols of phthalic acid).

There have been developed numerous techniques directed towards the preparation of carboxylated aromatic compounds, falling within the class defined by the formula (I).

Carboxylic arylacids and their esters have been described as being obtainable by the carboxylation reaction of aryl halides with carbon monoxide in the presence of a transition metal.

In general such reactions require the application of high pressures and temperatures; the severe operational conditions adversely affect the economic efficiency of the processes, thus considerably reducing their commercial value.

A variant of the above synthesis envisages the use of $Ni(CO)_4$ catalysts and of special aprotic dipolar solvents associated with basic compounds that will allow milder operational conditions.

The use of tetracarbonylnickel as a catalyst, due to its high degree of volatility and toxicity, is however disadvantageous and uneconomic; this process also involves the use of expensive solvents.

Similar disadvantages arise in respect of the known carbonylization process of aromatic halides in the presence of Pd catalysts with phosphinic ligands, at least from the point of view of economic efficiency.

Quite recently there has been described the use, for the above-mentioned carbonylization reaction, of aromatic halides, of catalytic systems based on cobalt complexes, on alcoholates and sodium hydride, or on carbonyl cobalt associated with ultraviolet radiation. It is, however, difficult, at present, to foresee practical application of such methods.

Thus, the aim of the present invention is that of providing a simple and economic catalytic process for the preparation of carboxylated aromatic compounds of formula (I), that is essentially free of the drawbacks above discussed.

The present invention provides a process for the preparation of carboxylated aromatic or hetero-aromatic compounds of formula (I), as previously defined, wherein an aromatic halide of formula (II):

$$Y'{-}Ar{-}X \qquad (II)$$

wherein Ar has the meaning previously defined, X is a halogen selected from Cl, Br and I, and Y′ is $-CH_2X$ or Y wherein X and Y have the meanings previously defined, is made to react with carbon monoxide in an alcoholic or hydro-alcoholic solvent and with an acidity acceptor compound, in the presence of a catalyst system comprising (a) a cobalt hydrocarbonyl salt or a precursor thereof, and, if required, (b) a halide of formula (III):

$$R'{-}X \qquad (III)$$

wherein R′ represents an alkyl group having up to 10 carbon atoms, a benzyl group or a group selected from:

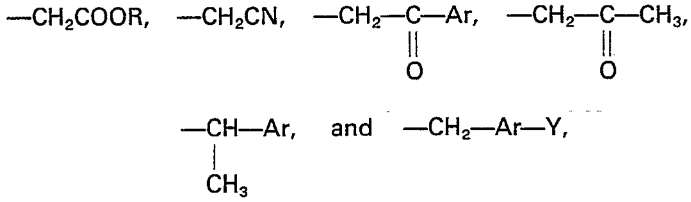

and

wherein Ar and R have the meanings previously defined, the presence of the said halide of formula (III) being unnecessary when the reacting aromatic halide of formula (II) belongs to the class of halides of formula (III), preferably under substantially atmospheric pressure, and at a temperature from about 20°C to 80°C.

According to the present invention, there is provided a process for the carbonylation of aromatic or hetero-aromatic halogenated substrates, under mild operational conditions, due to the use of an effective catalyst system.

The process according to the invention thus envisages the carbonylation reaction of an aromatic halogenated substrate of formula (II), as herein above defined, conducted in a (hydro)-alcoholic medium and in the presence of a basic acidity acceptor, and of a catalyst system formed of a cobalt hydrocarbonyl salt or a precursor thereof and of a halide of formula (III), as herein above defined, at a temperature from about 20°C to 80°C, under substantially atmospheric pressure.

Where Ar is a hetero-aromatic nucleus, there may be contained in it one or more of N, O and S.

Moreover, when more than one aromatic rings are present, they are associated with each other according to a condensed ring structure or to a ring structure bound through either a direct C—C bond or otherwise.

Finally, when in the starting compound or substrate of formula (II), besides halide X, there also is present a further halogen atom in the substituent group $Y'=CH_2X$ as herein defined, there are obtained the corresponding bi-carboxylated compounds of formula (I), wherein Y is $CH_2COOR$ and wherein the other symbols have the same meanings.

Depending on the operational conditions, from the reaction there will be obtained the acids and/or their alkaline salts and their esters.

From such alkaline salts, the corresponding acids may be obtained by displacement with mineral acids (HCl, $H_2SO_4$), according to conventional methods or by the hydrolysis of the esters.

By the term "precursor" as it appears in the present disclosure, there is meant one or more compounds which, under operational conditions envisaged by the process, give place to the above-mentioned cobalt hydro-carbonyl salt.

The reaction may be schematically represented through the following equations:

$$\text{(1)} \quad Ar{-}X+CO+2MOH \xrightarrow[\text{MOH/H}_2\text{O/alcohol}]{\text{Cat.}[Co(CO)_4]^-} Ar{-}COOM+MX+H_2O$$

$$\text{(2)} \quad Ar{-}X+CO+ROH+B \xrightarrow[\text{alcohol/B}]{\text{Cat.}[Co(CO_4)]^-} Ar{-}COOR+BHX,$$

wherein the symbols have the previously defined meanings and B is either an inorganic base or an amine base. From the hydroalcoholic mixture of reaction (1), it is possible to also obtain the carboxylic acid directly by acidification.

The reaction is carried out in the presence of a hydroalcoholic or alcoholic solvent medium, depending on whether there have to be prepared the alkali or alkaline-earth salts of (hetero)-aromatic acids and, thus, their corresponding free acids by means for example of displacement with mineral acids or extraction, according to reaction (1), or, according to reaction (2), whether it is desired to prepare the esters of the aromatic acids.

Effective solvents are the low aliphatic alcohols, having up to 4 carbon atoms, optionally in admixture with water. Particularly suitable are methyl and ethyl alcohols.

As stated above, the carbonylation reaction is conducted in the presence of acidity acceptor compounds that develop during the reaction itself. Suitable acidity acceptors in general are both inorganic and organic basic substances. More particularly, suitable inorganic bases are carbonates, oxides and hydroxides of alkali and alkaline-earth metals, particularly Na, K, Ca, and Mg, while suitable organic bases are tertiary amines.

The preferred inorganic bases are potassium and sodium carbonates, calcium and magnesium oxides, and sodium and potassium hydroxides, while the preferred organic bases are triethylamine, tetramethylguanidine and mixtures thereof.

The reaction is carried out in the presence of a catalyst system comprising, as herein above indicated:

(a) a cobalt hydrocarbonyl salt and

(b) a R'—X halide, as herein above defined.

More particularly, the cobalt hydrocarbonyl salts (a) are preferably of the formula:

$$Me^{n+}[Co(CO)_4]_n \quad (IV)$$

wherein Me represents a cation of a metal having a valency n, such as the alkali metals (preferably Na, K, Li) or cobalt, iron or manganese, all known and preparable according to conventional techniques of the art. Preferred salts are sodium, cobalt, manganese and iron salts of formula (IV).

There may also be used precursors of the above-mentioned salts. For example, the alkali salt of cobalt hydrocarbonyl may be obtained *in situ* from $Co_2(CO)_8$ under the existing reaction conditions.

Alternatively, separately, the cobalt hydrocarbonyl salt may be prepared from a cobalt salt such as a chloride, sulphate or bromide, a powdery Fe-Mn alloy (containing about 80% of Mn) and from a sulphurated promoter, preferably an alkaline sulphide or thiosulphate, in methyl or ethyl alcohol, at a carbon monoxide pressure of from 1 to 20 atm. and at a temperature of from about 10° to 80°C, preferably from about 25° to 35°C.

The concentration of the cobalt salt in the solution is preferably from 0.3 to 1 mol/litre. For each mol of cobalt there are used from 1 to 2 mols of Mn in the form of an Fe/Mn alloy. This Fe/Mn alloy is preliminary ground so that it may pass through a sieve of 5000 mesh/sq. cm.

Preferred sulphurated promoters are sodium sulphide and thiosulphate, and are used in quantities of from 0.01 to 0.1 mols per mol of cobalt salt.

The alcoholic mixture containing the cobalt salt, the alloy and the sulphurated promoter in the alcoholic solvent is kept under a CO atmosphere under vigorous stirring, for a time sufficient to complete the absorption of the CO, a time that will amount to at least 2—3 hours. In this way there may be obtained the Mn and/or Fe salts of the cobalt hydrocarbonyl.

The decanted solution may be introduced directly into the reactor, thus obtaining the catalyst of the process of the invention in an alcoholic solution.

Alternatively, still separately, the sodium salt from $Co_2(CO)_8$ may be obtained by reduction with a sodium amalgam in an ether solvent (tetrahydrofuran).

In its turn, $Co_2(CO)_8$ is prepared, for instance, from $CoCO_3$ under CO and hydrogen pressure in petroleum ether.

The halogenated component (b) of formula (III) of the catalyst system, when R' is a $—CH_2—Ar—Y$ group wherein Y is Cl, Br or I, may coincide with one of the possible substrates of formula (II), and *vice versa*, when in formula (II) Y' is a $—CH_2X$ group wherein X is Cl, Br or I, compound (II) itself may coincide with one of the possible components (b) of formula (III) of the catalytic system.

In such instances, it is clearly possible to operate optionally with a catalytic system reduced to the component (a), that is to the salt of the cobalt hydrocarbonyl; in this case, component (b) consists of the halogenated substrate (II) itself.

The molar ratio between the starting aromatic halide (II) and the component (a) of the catalyst system, consisting of the cobalt hydrocarbonyl salt (IV), is preferably from 5:1 to 150:1, more preferably from 10:1 to 100:1, while the molar ratio between the starting aromatic halide (II) and the component (b) of the catalyst system, consisting of the halide of formula (III), is preferably from 1:1 to 50:1, more preferably from 10:1 to 30:1, per mol of substrate (II).

The acidity acceptor compound may be suitably introduced in a quantity which is substantially stoichiometric with respect to the equilibrium of either reaction (1) or (2); an excess of up to about 50% by weight is compatible with the reaction.

The carbon monoxide is admixed at a substantially atmospheric pressure; higher values are compatible although not necessary.

The admixture of the halide of formula (III), component (b) of the catalyst system, may be carried out in a single step, or gradually during the course of steady reaction.

The reaction will in general be completed within a period of time of about 5 to 24 hours, depending on the operational conditions of for example temperature, type of starting substrate (II), and type of components (a) and (b) of the catalytic system.

Suitable starting aromatic halides(II) are 2 - chloronaphthalin, 2 - chlorothiophene, 2 - chloromethyl - 5 - chlorothiophene, 2 - chloromethyl - 5 - bromothiophene, bromobenzene, 4 - chlorobenzonitrile, 1 - chloronaphthalin, 4 - bromotoluene, 4 - bromoanisol, p - dibromobenzene, 4 - chloro - bromobenzene, methyl - 4 - chlorobenzoate, 2 - bromothiophene, 2 - bromoacetonanilide, 4 - chlorobenzotrifluoride, 2 - bromoaniline, 3 - bromopyridine, p - bromobenzylchloride, and 3 - bromofuran.

Suitable halides of formula (III), as components (b) of the catalytic system, are 2 - chloromethyl - 5 - chlorothiophene, 2 - chloromethyl - 5 - bromothiophene, benzyl chloride, methyl chloroacetate, ethyl chloroacetate, α - chlorophenylethane, α - bromophenylethane, chloroacetonitrile, chloroacetophenone, methyl iodide, 1 - bromooctane, 1 - chlorooctane, p - chlorobenzylchloride, chloromethylthiophene, chloroethylnaphthalin, and chloromethylfuran.

Suitable salts of the cobalt hydrocarbonyl of formula (IV), as components (a) of the catalyst system, are the sodium salt of cobalt hydrocarbonyl, $NaCo(CO)_4$, and the cobalt salt $Co[Co(CO)_4]_2$. Suitable precursors are cobalt octacarbonyl and cobalt chloride associated with powdery iron-manganese and sodium thiosulphate, as above.

By means of the present invention there may be obtained directly from the reaction mixture the esters or the salts of the acids in an alcoholic or hydroalcoholic medium. More particularly, the alkaline salt or the esters are separated in the form of respectively a free acid or ester, according to known or substantially conventional methods. For example, in the case there have been obtained the alkali or alkaline-earth salts (reaction 1), water is added, the solution is acidified with a mineral acid (e.g. HCl) and the whole is then extracted with a solvent (e.g. ether). The organic phase is, in its turn, extracted with sodium bicarbonate, acidified again and extracted with ether which, after evaporation, yields the acid which may then be subjected to purification.

In the case of the esters (reaction 2), the reaction product is diluted with water, then acidified and finally extracted with ether. After evaporation of the ether, the ester is isolated as such according to known techniques, or it may be saponified in either an acid or basic medium, in order to obtain the corresponding acid.

The process according to the invention may be suitably conducted in the following way.

Into a reactor, fitted with a stirrer, a thermometer and a cooler, there are introduced, under an atmosphere of carbon monoxide at atmospheric pressure, the (hydro)-alcoholic solvent, the acidity acceptor, the salt of the cobalt hydrocarbonyl (IV) or its precursor, as component (a) of the catalyst system, and the aromatic halide of formula (II), as the chosen substrate for the carbonylation.

Thereupon there is admixed the component (b) of the catalyst system, i.e. the halide of formula (III), if required, and differing from compound (II), as herein above explained.

The reaction mixture is then brought up to the desired temperature under stirring, while maintaining the CO head, for the required time.

At the end of the reaction, shown by the stopping of the absorption of the CO, the product is separated according to a procedure previously described, according to a substantially conventional technique.

The process, due to the mild and simple operational conditions, is particularly convenient, in particular for the high selectivity that may be achieved with respect to the desired product.

The invention will be further described with reference to the following illustrative Examples.

Example 5 is given for comparative purposes in the absence of component (b) of the catalytic system, of formula (III).

Example 1:

Into a 50 cc reactor, fitted with a magnetic stirrer, a thermometer and a water cooler, under a CO head, there were introduced 0.4 grams of cobalt octacarbonyl.

25 cc of methanol, 5.10 g (m.mols 37) of potassium carbonate, 0.40 g (m.mols 3.95) of methyl chloroacetate and 4.4 grams (m.mols 27) of 2-chloronaphthalin were then added.

The reaction temperature was then brought to 60°C and was kept at this level for 6 hours, under stirring. Thereby there were absorbed 530 cc of CO.

The reaction mixture was then saponified by adding 3 grams of sodium hydroxide and 20 cc of water, and keeping it boiling for about 2 hours.

After cooling, there was added water and the whole was then acidified with concentrated HCl and extracted with ether.

The organic phase was extracted with a saturated solution of sodium bicarbonate and the aqueous layer was then re-acidified, and finally extracted with ether.

By evaporation under vacuum of the solvent, there were obtained 4.2 grams (m.mols 24.4) of 2-naphthoic acid. The yield was 91%, calculated with reference to the starting 2-chloronaphthalin.

Examples 2—40:

By operating as described in Example 1, there were obtained the carboxylated products given in the following Table, which Table also gives the corresponding operational conditions.

In Examples 2, 3 and 4 and in Examples 36 and 39, respectively carried out in a hydroalcoholic medium (2, 3, 4) or in the presence of strong bases (36 and 39), the saponification phase was not effected because the reaction lead straight to the alkaline salt of the carboxylic salt.

The recorded values refer to the quantities expressed in the units as indicated at the head of the Table.

TABLE

| No. | Ar X | g | Solvent | cc | Base | g | R'X | g | Catalyst | g | T C° | t(h) | Product (yield) | % |
|---|---|---|---|---|---|---|---|---|---|---|---|---|---|---|
| 2 | 2-chloromethyl-5-chlorothiophene | 2.8 | $CH_3OH/H_2O$ | 12/0.4 | CaO | 2.5 | — | — | $Co_2(CO)_8$ | 0.34 | 35 | 8 | 5-carboxy-2-thienylacetic acid | 33 |
| 3 | 2-chloromethyl-5-bromothiophene | 4.05 | $CH_3OH/H_2O$ | 12/0.4 | CaO | 2.5 | — | — | $Co_2(CO)_8$ | 0.34 | 22 | 8 | 5-carboxy-2-thienylacetic acid | 48 |
| 4 | 2-chlorothiophene | 3.2 | $CH_3CH/H_2O$ | 25/1 | CaO | 6 | benzyl chloride | 1.2 | $Co_2(CO)_8$ | 0.9 | 35 | 8 | 2-thiophen-carboxylic acid | 65 |
| 5 | 2-chlorothiophene | 6.4 | $CH_3OH$ | 25 | $K_2CO_3$ | 14.8 | — | — | $Co_2(CO)_8$ | 0.9 | 35 | 24 | 2-thiophen-carboxylic acid | 0 |
| 6 | 2-chlorothiophene | 6.4 | $CH_3OH$ | 25 | $K_2CO_3$ | 14.8 | benzyl chloride | 1 | $Co_2(CO)_8$ | 0.9 | 35 | 24 | 2-thiophen-carboxylic acid | 72 |
| 7 | bromobenzene | 8.5 | $CH_3OH$ | 25 | $K_2CO_3$ | 14.8 | benzyl chloride | 1 | $Co_2(CO)_8$ | 0.9 | 35 | 24 | benzoic acid | 58 |
| 8 | 4-chlorobenzo-nitrile | 7.4 | $CH_3OH$ | 35 | $K_2CO_3$ | 10.2 | benzyl chloride | 2 | $Co_2(CO)_8$ | 0.9 | 45 | 24 | 4-cyanobenzoic acid | 37 |
| 9 | bromobenzene | 8.5 | $CH_3OH$ | 25 | $Na_2CO_3$ | 8 | benzyl chloride | 1 | $Co_2(CO)_8$ | 0.9 | 37 | 24 | benzoic acid | 30 |
| 10 | bromobenzene | 4.25 | $CH_3OH$ | 25 | $K_2CO_3$ | 5.1 | methyl chloroacetate | 0.4 | $Co_2(CO)_8$ | 0.56 | 60 | 24 | benzoic acid | 88 |
| 11 | 2-chlorothiophene | 3.2 | $C_2H_5OH$ | 25 | $K_2CO_3$ | 5.1 | ethyl chloroacetate | 1.5 | $Co_2(CO)_8$ | 0.45 | 45 | 24 | 2-thiophen-carboxylic acid | 61 |

TABLE (Continued)

| No. | Ar X | g | Solvent | cc | Base | g | R'X | g | Catalyst | g | T C° | t(h) | Product (yield) | % |
|---|---|---|---|---|---|---|---|---|---|---|---|---|---|---|
| 12 | 2-chlorothiophene | 3.2 | $CH_3OH$ | 15 | $K_2CO_3$ | 5.1 | methyl chloroacetate | 0.22 | $Co_2(CO)_8$ | 0.28 | 60 | 21 | 2-thiophen-carboxylic acid | 98 |
| 13 | 4-chloro-naphthalin | 4.4 | $CH_3OH$ | 25 | $K_2CO_3$ | 5.1 | methyl chloroacetate | 0.4 | $Co_2(CO)_8$ | 0.52 | 60 | 8 | 1-naphthoic acid | 82 |
| 14 | bromobenzene | 4.25 | $CH_3OH$ | 25 | $K_2CO_3$ | 5.1 | α-chloro-phenylethane | 0.86 | $Co_2(CO)_8$ | 0.49 | 45 | 24 | benzoic acid | 20 |
| 15 | bromobenzene | 4.25 | $CH_3OH$ | 25 | $K_2CO_3$ | 5.1 | β-bromo-phenylethane | 0.73 | $Co_2(CO)_8$ | 0.53 | 45 | 7 | benzoic acid | 20 |
| 16 | bromobenzene | 4.25 | $CH_3OH$ | 25 | $K_2CO_3$ | 5.1 | methyl chloroacetate | 0.4 | $NaCo(CO)_4$ | 0.46 | 60 | 25 | benzoic acid | 85 |
| 17 | 4-bromotoluene | 4.6 | $CH_3OH$ | 25 | $K_2CO_3$ | 5.1 | methyl chloroacetate | 0.4 | $Co_2(CO)_8$ | 0.5 | 60 | 23 | p-toluic acid | 70 |
| 18 | 4-bromoanysol | 5 | $CH_3OH$ | 25 | $K_2CO_3$ | 5.1 | methyl chloroacetate | 0.4 | $Co_2(CO)_8$ | 0.5 | 60 | 21 | 1-methoxy benzoic acid | 73 |
| 19 | 2-chloro-thiophene | 3.2 | $CH_3OH$ | 25 | $K_2CO_3$ | 5.1 | methyl chloroacetate | 0.4 | $NaCoCO_4$ | 0.49 | 60 | 18 | 2-thiophen-carboxylic acid | 95 |
| 20 | p-dibromobenzene | 6.4 | $CH_3OH$ | 25 | $K_2CO_3$ | 10.25 | methyl chloroacetate | 0.4 | $Co_2(CO)_8$ | 0.5 | 60 | 25 | terephthalic acid | 98 |
| 21 | 4-chloro-bromobenzene | 5.2 | $CH_3OH$ | 25 | $K_2CO_3$ | 10.25 | methyl chloroacetate | 0.4 | $Co_2(CO)_8$ | 0.5 | 60 | 23 | 4-chlorobenzoic acid | 80 |
| 22 | bromobenzene | 4.25 | $CH_3OH$ | 20 | $K_2CO_3$ | 5.1 | methyl chloroacetate | 0.4 | catalyst solution* | (cc 5) | 60 | 22 | benzoic acid | 80 |
| 23 | 2-chloro-thiophene | 3.2 | $CH_3OH$ | 25 | $K_2CO_3$ | 5.1 | chloro-acetonitrile | 0.3 | $Co_2(CO)_8$ | 0.5 | 60 | 20 | 2-thiophen-carboxylic acid | 70 |

TABLE (Continued)

| No. | Ar X | g | Solvent | cc | Base | g | R'X | g | Catalyst | g | T C° | t(h) | Product (yield) | % |
|---|---|---|---|---|---|---|---|---|---|---|---|---|---|---|
| 24 | 2-chlorothiophene | 3.2 | $CH_3OH$ | 20 | $K_2CO_3$ | 5.1 | methyl chloroacetate | 0.4 | catalyst solution* | (cc 5) | 60 | 21 | 2-thiophen-carboxylic acid | 90 |
| 25 | bromobenzene | 4.25 | $CH_3OH$ | 25 | $K_2CO_3$ | 5.1 | chloroaceto-phenone | 0.5 | $Co_2(CO)_8$ | 0.5 | 60 | 22 | benzoic acid | 20 |
| 26 | methyl 4-chloro-benzoate | 4.6 | $CH_3OH$ | 25 | $K_2CO_3$ | 5.1 | methyl chloroacetate | 0.4 | $Co_2(CO)_8$ | 0.5 | 60 | 7 | terephthalic acid | 15 |
| 27 | 2-chlorothiophene | 3.2 | $CH_3OH$ | 25 | $K_2CO_3$ | 5.1 | methyl iodide | 0.56 | $Co_2(CO)_8$ | 0.5 | 37 | 24 | 2-thiophen-carboxylic acid | 60 |
| 28 | 2-bromothiophene | 4.4 | $CH_3OH$ | 25 | $K_2CO_3$ | 5.1 | methyl chloroacetate | 0.4 | $Co_2(CO)_8$ | 0.5 | 60 | 16 | 2-thiophen-carboxylic acid | 95 |
| 29 | bromobenzene | 4.25 | n-propanol/ $H_2O$ | 25/1 | $K_2CO_3$ | 5.1 | methyl chloroacetate | 0.8 | $Co_2(CO)_8$ | 0.5 | 60 | 20 | benzoic acid | 35 |
| 30 | bromobenzene | 4.25 | $CH_3OH$ | 25 | triethyl-amine | 3.7 | methyl chloroacetate | 0.4 | $Co_2(CO)_8$ | 0.58 | 60 | 23 | benzoic acid | 30 |
| 31 | bromobenzene | 4.25 | $CH_3OH$ | 25 | tetramethyl-guanidine | 4.2 | methyl chloroacetate | 0.4 | $Co_2(CO)_8$ | 0.52 | 60 | 22 | benzoic acid | 30 |
| 32 | 2-bromo-acetanilide | 3.9 | $CH_3OH$ | 20 | $K_2CO_3$ | 3.5 | methyl chloroacetate | 0.3 | $Co_2(CO)_8$ | 0.5 | 60 | 21 | anthranylic acid | 40 |
| 33 | 4-chlorobenzotri-fluoride | 4.9 | $CH_3OH$ | 25 | $K_2CO_3$ | 5.1 | methyl chloroacetate | 0.86 | $Co_2(CO)_8$ | 0.5 | 60 | 22 | 4-trifluoromethyl-benzoic acid | 36 |
| 34 | bromobenzene | 4.25 | $CH_3OH$ | 25 | $K_2CO_3$ | 5.1 | 1-bromooctane | 0.77 | $Co_2(CO)_8$ | 0.62 | 60 | 7 | benzoic acid | 97 |

TABLE (Continued)

| No. | Ar X | g | Solvent | cc | Base | g | R'X | g | Catalyst | g | T C° | t(h) | Product (yield) | % |
|---|---|---|---|---|---|---|---|---|---|---|---|---|---|---|
| 35 | 2-chlorothiophene | 3.2 | $CH_3OH$ | 25 | $K_2CO_3$ | 5.1 | 1-chloro-octane | 0.59 | $Co_2(CO)_8$ | 0.5 | 60 | 22 | 2-thiophen-carboxylic acid | 60 |
| 36 | 2-chlorothiophene | 3.2 | $CH_3OH$ | 25 | NaOH | 2.5 | benzyl chloride | 0.5 | $Co_2(CO)_8$ | 0.5 | 30 | 6 | 2-thiophen-carboxylic acid | 86 |
| 37 | bromobenzene | 4.25 | $CH_3OH$ | 25 | $K_2CO_3$ | 5.1 | p-chlorobenzene chloride | 0.64 | $Co_2(CO)_8$ | 0.5 | 35 | 20 | benzoic acid | 70 |
| 38 | 2-bromoaniline | 4.65 | $CH_3OH$ | 25 | $K_2CO_3$ | 5.1 | methyl chloroacetate | 0.4 | $Co_2(CO)_8$ | 0.5 | 60 | 27 | anthranylic acid | 20 |
| 39 | 3-bromopyridine | 4.27 | $CH_3OH$ | 25 | NaOH<br>$Net_3$ | 3<br>0.6 | benzyl chloride | 0.5 | $Co_2(CO)_8$ | 0.5 | 35 | 20 | nicotinic acid | 55* |
| 40 | 3-bromofurane | 3.5 | $CH_3OH$ | 25 | $K_2CO_3$ | 5 | methyl chloroacetate | 0.35 | $Co_2(CO)_8$ | 0.4 | 60 | 20 | 3-furan carboxylic acid | 93 |

* The catalyst solution was prepared from 20 g of $CoCl_2 . 6H_2O$, 0.6 g of $Na_2S . 9H_2O$ and 1.5 g of $Na_2S_2O_3 . 5H_2O$ in 180 ml of methanol, under stirring and in a CO atmosphere at 30°C, there were introduced 1 & g of a Mn/Fe alloy, ground in such a way as to pass through a 10,000 mesh/sq. cm sieve. The stirring was maintained for 3 hours. The solution thus obtained was ready for use.

** The acid was recovered by extraction in soxlet with ethyl acetate of the aqueous solution at the isoelectric point (pH 3.42 at 25°C).

Example 41:

Into a 50 cc reactor provided with a magnetic stirrer, a thermometer and a water cooler, there were introduced, under a CO head, 0.5 g of cobalt octacarbonyl.

To this were then added 25 cc of methanol, 6.0 g (43 m.mols) of potassium carbonate, 0.4 g (3.95 m.mols) of methyl chloroacetate and 3.2 g (27 m.mols) of 2-chlorothiophene.

This mixture was then brought to a temperature of 60°C and was maintained at this value, under stirring, over 7 hours. Thereby there were absorbed 500 cc of CO.

After cooling down of the reaction mixture, there was added water, after which it was acidified with concentrated HCl and then extracted with ether.

The organic phase, after anhydrification and evaporation of the solvent, was distilled under vacuum. Thereby there were obtained 3 g (21.1 m.mols) of methyl thiophenecarboxylate (boiling point: 84—86/15 mmHg), in a yield equal to 78.1%.

**Claims**

1. A process for the preparation of aromatic or hetero-aromatic carboxylated compounds of formula (I):

$$Y—Ar—\underset{\underset{O}{\|}}{C}—OR \qquad (I)$$

wherein Ar represents an aromatic nucleus having one or more benzenic rings optionally (poly)-condensed, or a hetero-aromatic nucleus optionally condensed, and wherein Y represents a hydrogen atom, a halogen selected from Cl, Br and I, an alkyl, alkoxy, or trifluoroalkyl group having up to 4 carbon atoms, an ester group, an aryloxyl group, a nitrile group, an amide group, an acetamide group, an amine group, or an acyl R″—CO— group wherein R″ represents an alkyl or aryl group, or Y represents a $—CH_2COOR$ group wherein R represents a hydrogen atom, an alkyl group having up to 4 carbon atoms, or wherein R represents an alkali- or an alkaline-earth metal, characterized in that an aromatic halide of formula (II):

$$Y'—Ar—X \qquad (II)$$

wherein Ar has the meaning previously defined, X is a halogen selected from Cl, Br and I, and Y′ is $—CH_2X$ or Y wherein X and Y have the meanings previously defined, is made to react with carbon monoxide in an alcoholic or hydro-alcoholic solvent and with an acidity acceptor compound, in the presence of a catalyst system comprising: (a) a cobalt hydrocarbonyl salt or a precursor thereof, and, if required, (b) a halide of formula (III):

$$R'—X \qquad (III)$$

wherein R′ represents an alkyl group having up to 10 carbon atoms, a benzyl group or a group selected from

$$—CH_2COOR,\quad —CH_2CN,\quad —CH_2—\underset{\underset{O}{\|}}{C}—Ar,\quad —CH_2—\underset{\underset{O}{\|}}{C}—CH_3,$$

$$—\underset{\underset{CH_3}{|}}{CH}—Ar, \quad \text{and} \quad —CH_2—Ar—Y,$$

where Ar and R have the meanings previously defined, the presence of the said halide of formula (III) being unnecessary when the reacting aromatic halide of formula (II) belongs to the class of halides of formula (III), and at a temperature from about 20° to 80°.

2. A process as claimed in Claim 1, characterized in that the solvent is selected from aliphatic alcohols having up to 4 carbon atoms, optionally in admixture with water.

3. A process as claimed in Claim 2, characterized in that the solvent is methyl alcohol or ethyl alcohol.

4. A process as claimed in any of Claims 1 to 3, characterized in that the acidity acceptor compound is a basic inorganic or organic substance.

5. A process as claimed in Claim 4, characterized in that the basic substance is selected from carbonates, oxides and hydroxides of alkali and alkaline-earth metals and from tertiary amines.

6. A process as claimed in Claim 5, characterized in that the basic substance is selected from carbonates, oxides and hydroxides of Na, K, Ca and Mg and from triethylamine, tetramethyl-guanidine and mixtures thereof.

7. A process as claimed in any of Claims 1 to 6, characterized in that component (a) of the catalyst system is a salt of the cobalt hydrocarbonyl of the formula (IV):

$$Me^{n+}[Co(CO)_4]_n \qquad (IV)$$

wherein Me represents a metal of valency n, or a precursor of a said salt.

8. A process as claimed in Claim 7, characterized in that the metal Me is selected from Na, K, Li, Co, Mn and Fe.

9. A process as claimed in Claim 7 or 8, characterized in that the cobalt hydrocarbonyl salt (a) is selected from sodium, cobalt, iron and manganese salts.

10. A process as claimed in any of Claims 7 to 9, characterized in that the cobalt hydrocarbonyl salt (a) is prepared from a cobalt salt, from a powdery Fe-Mn alloy containing about 80% of manganese and from a sulphurated promoter, in methyl alcohol or ethyl alcohol, by reaction with CO at a pressure of from 1 to 20 atmospheres and at a temperature of from 10° to 80°C.

11. A process as claimed in Claim 10, characterized in that the said cobalt salt is a chloride, bromide or sulphate.

12. A process as claimed in Claim 10 or 11, characterized in that the said sulphurated promoter is an alkaline sulphide or thiosulphate.

13. A process as claimed in any of Claims 10 to 12, characterized in that the said reaction temperature is from 25° to 35°C.

14. A process as claimed in any of Claims 7 to 9, characterized in that the alkaline salt of cobalt hydrocarbonyl (a) is prepared *in situ* from $Co_2(CO)_8$ in the alkaline medium of the reaction.

15. A process as claimed in any of Claims 1 to 14, characterized in that the molar ratio between the starting aromatic halide (II) and the component (a) of the catalyst system is from 5:1 to 150:1.

16. A process as claimed in Claim 15, characterized in that the molar ratio between the starting aromatic halide (II) and the component (a) of the catalyst system is from 10:1 to 100:1.

17. A process as claimed in any of Claims 1 to 16, characterized in that the molar ratio between the starting aromatic halide (II) and the component (b) of the catalyst system is from 1:1 to 50:1.

18. A process as claimed in Claim 17, characterized in that the molar ratio of the starting aromatic halide (II) and the component (b) of the catalyst system is from 10:1 to 30:1.

19. A process as claimed in any of Claims 1 to 18, characterized in that the acidity acceptor compound is present in a quantity which is substantially stoichiometric with respect to the reaction equilibrium, up to an excess of about 50% by weight over the said quantity.

20. A process as claimed in any of Claims 1 to 19, characterized in that the starting aromatic halide is selected from 2 - chloronaphthalin, 2 - chlorothiophene, 2 - chloromethyl - 5 - chlorothiophene, 2 - chloromethyl - 5 - bromothiophene, bromobenzene, 4 - chlorobenzonitrile, 1 - chloronaphthalin, 4 - bromotoluene, 4 - bromoanisol, p - dibromobenzene, 4 - chloro - bromobenzene, methyl 4 - chlorobenzoate, 2 - bromothiophene, 2 - bromoacetoanilide, 4 - chlorobenzotrifluoride, 2 - bromoaniline, 3 - bromopyridine, p - bromobenzylchloride, and 3 - bromofuran.

21. A process as claimed in any of Claims 1 to 20, characterized in that the component (b) of the catalyst system is selected from 2 - chloro - methyl - 5 - chlorothiophene, 2 - chloromethyl - 5 - bromothiophene, benzyl chloride, methyl chloroacetate, ethyl chloroacetate, α - chlorophenylethane, α - bromophenylethane, chloroacetonitrile, chloroacetophenone, methyl iodide, 1 - bromooctane, 1 - chlorooctane, p - chlorobenzylchloride, chloromethylthiophene, chloromethylnaphthalin, and chloromethylfuran.

22. A process as claimed in any of Claims 1 to 21, characterized in that the carboxylated aromatic compound of formula (I) is a bi-carboxylated compound when in the starting halide (II) there is present a $—CH_2X$ group in which X is selected from Cl, Br and I.

23. A process as claimed in any of Claims 1 to 22, characterized in that in the presence of an hydro-alcoholic solvent medium there are substantially obtained the alkali- or alkaline-earth salts of the carboxylated aromatic compounds of formula (I).

24. A process as claimed in any of Claims 1 to 22, characterized in that in the presence of an alcoholic solvent medium there are substantially obtained the alkyl esters of the carboxylated aromatic compounds of formula (I).

25. A process as claimed in any of Claims 1 to 24, wherein the said reaction is carried out under substantially atmospheric pressure.

**Patentansprüche**

1. Verfahren zur Herstellung von aromatischen oder heteroaromatischen carboxylierten Verbindungen der Formel (I)

$$Y—Ar—\underset{\underset{O}{\|}}{C}—OR \qquad (I)$$

worin Ar für einen aromatischen Kern mit einem oder mehreren, gegebenenfalls (poly)-kondensierten, Benzolringen oder einen heteroaromatischen, gegebenenfalls kondensierten, Kern steht, und

worin Y ein Wasserstoffatom, ein Halogen, ausgewählt aus Cl, Br und J, eine Alkyl-, Alkoxy- oder Trifluoralkylgruppe mit bis zu 4 Kohlenstoffatomen, eine Estergruppe, eine Aryloxygruppe, eine Nitrilgruppe, eine Amidgruppe, ein Acetamidgruppe, eine Amingruppe oder eine Acyl —(R″—CO—) Gruppe, worin R″ eine Alkyl- oder Arylgruppe ist, bedeutet, oder Y steht für eine $-CH_2COOR$ Gruppe, worin R ein Wasserstoffatom, eine Alkylgruppe mit bis zu 4 Kohlenstoffatomen ist oder worin R ein Alkali- oder Erdalkalimetall ist, dadurch gekennzeichnet, daß ein aromatisches Halogenid der Formel (II):

$$Y'-Ar-X \qquad (II)$$

worin Ar die oben definierte Bedeutung hat, X ein Halogen, ausgewählt aus Cl, Br und J ist, und Y′ $-CH_2X$ oder Y bedeutet, worin X und Y die oben definierte Bedeutung haben, mit Kohlenmonoxid in einem alkoholischen oder hydro-alkoholischen Lösungsmittel und mit einem Säureakzeptor in Anwesenheit eines Katalysatorsystems umgesetzt wird, das (a) ein Kobalthydrocarbonylsalz oder einen Vorläufer desselben und, gegebenenfalls, (b) ein Halogenid der Formel (III):

$$R'-X \qquad (III)$$

umfaßt, worin R′ eine Alkylgruppe mit bis zu 10 Kohlenstoffatomen, eine Benzylgruppe oder eine Gruppe, ausgewählt aus

$-CH_2COOR$, $-CH_2CN$, 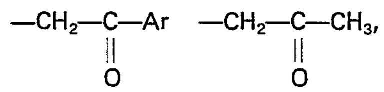

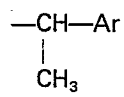 und $-CH_2-Ar-Y$,

Ar und R die oben definierte Bedeutung haben, ist, wobei die Anwesenheit des Halogenids der Formel (III) unnötig ist, wenn das umzusetzende aromatische Halogenid der Formel (II) zur Klasse der Halogenide der Formel (III) gehört, und wobei die Reaktion bei einer Temperatur von etwa 20 bis 80° durchgeführt wird.

2. Verfahren nach Anspruch 1, dadurch gekennzeichnet, daß das Lösungsmittel ausgewählt ist aus aliphatischen Alkoholen mit bis zu 4 Kohlenstoffatomen, wahlweise in Mischung mit Wasser.

3. Verfahren nach Anspruch 2, dadurch gekennzeichnet, daß das Lösungsmittel Methylalkohol oder Ethylalkohol ist.

4. Verfahren nach einem der Ansprüche 1 bis 3, dadurch gekennzeichnet, daß die Säureakzeptorverbindung eine basische anorganische oder organische Substanz ist.

5. Verfahren nach Anspruch 4, dadurch gekennzeichnet, daß die basische Substanz ausgewählt ist aus Carbonaten, Oxiden und Hydroxiden von Alkali- und Erdalkalimetallen und tertiären Aminen.

6. Verfahren nach Anspruch 5, dadurch gekennzeichnet, daß die basische Substanz ausgewählt ist aus Carbonaten, Oxiden und Hydroxiden von Na, K, Ca und Mg und von Triethylamin, Tetramethylguanidin und Mischungen derselben.

7. Verfahren nach einem der Ansprüche 1 bis 6, dadurch gekennzeichnet, daß die Komponente (a) des Katalysatorsystems ein Salz des Kobalthydrocarbonyls der Formel (IV) ist:

$$Me^{n+}[Co(CO)_4]_n \qquad (IV)$$

worin Me ein Metall der Wertigkeit n bedeutet oder ein Vorläufer dieses Salzes ist.

8. Verfahren nach Anspruch 7, dadurch gekennzeichnet, daß das Metall Me ausgewählt ist aus Na, K, Li, Co, Mn und Fe.

9. Verfahren nach Anspruch 7 oder 8, dadurch gekennzeichnet, daß das Kobalthydrocarbonylsalz (a) ausgewählt ist aus Natrium-, Kobalt-, Eisen- und Mangansalzen.

10. Verfahren nach einem der Ansprüche 7 bis 9, dadurch gekennzeichnet, daß das Kobalthydrocarbonylsalz (a) aus einem Kobaltsalz, aus einer pulverförmigen, etwa 80% Mangan enthaltenden Fe-Mn Legierung und aus einem sulfurierten Promotor in Methylalkohol oder Ethylalkohol durch Reaktion mit CO bei einem Druck von 1 bis 20 at und bei einer Temperatur von 10 bis 80°C hergestellt ist.

11. Verfahren nach Anspruch 10, dadurch gekennzeichnet, daß das Kobaltsalz ein Chlorid, Bromid oder Sulfat ist.

12. Verfahren nach Anspruch 10 oder 11, dadurch gekennzeichnet, daß der sulfurierte Promotor ein Alkali-Sulfid oder Thiosulfat ist.

13. Verfahren nach einem der Ansprüche 10 bis 12, dadurch gekennzeichnet, daß die Reaktionstemperatur 25 bis 35°C beträgt.

14. Verfahren nach einem der Ansprüche 7 bis 9, dadurch gekennzeichnet, daß das Alkalisalz des Kobalthydrocarbonyls (a) in situ aus $Co_2(CO)_8$ im alkalischen Reaktionsmedium hergestellt wird.

15. Verfahren nach einem der Ansprüche 1 bis 14, dadurch gekennzeichnet, daß das molare Verhältnis zwischen dem aromatischen Ausgangshalogenid (II) und der Komponente (a) des Katalysatorsystems von 5:1 bis 150:1 beträgt.

16. Verfahren nach Anspruch 15, dadurch gekennzeichnet, daß das molare Verhältnis zwischen dem aromatischen Ausgangshalogenid (II) und der Komponente (a) des Katalysatorsystems von 10:1 bis 100:1 beträgt.

17. Verfahren nach einem der Ansprüche 1 bis 16, dadurch gekennzeichnet, daß das molare Verhältnis zwischen dem aromatischen Ausgangshalogenid (II) und der Komponente (b) des Katalysatorsystems von 1:1 bis 50:1 beträgt.

18. Verfahren nach Anspruch 17, dadurch gekennzeichnet, daß das molare Verhältnis des aromatischen Ausgangshalogenids (II) und der Komponente (b) des Katalysatorsystems von 10:1 bis 30:1 beträgt.

19. Verfahren nach einem der Ansprüche 1 bis 18, dadurch gekennzeichnet, daß die Säureakzeptorverbindung in einer Menge anwesend ist, die in Bezug auf das Reaktionsgleichgewicht praktisch stöchiometrisch ist, bis zu einem Überschuß von etwa 50 Gew.-% über diese Menge.

20. Verfahren nach einem der Ansprüche 1 bis 19, dadurch gekennzeichnet, daß das aromatische Ausgangshalogenid ausgewählt ist aus 2-Chlornaphthalin, 2-Chlorthiophen, 2-Chlormethyl-5-chlorthiophen, 2-Chlormethyl-5-bromthiophen, Brombenzol, 4-Chlorbenzonitril, 1-Chlornaphthalin, 4-Bromtoluol, 4-Bromanisol, p-Dibrombenzol, 4-Chlorbrombenzol, Methyl-4-chlorbenzoat, 2-Bromthiophen, 2-Bromacetanilid, 4-Chlorbenzotrifluorid, 2-Bromanilin, 3-Brompyridin, p-Brombenzylchlorid und 3-Bromfuran.

21. Verfahren nach einem der Ansprüche 1 bis 20, dadurch gekennzeichnet, daß die Komponente (b) des Katalysatorsystems ausgewählt ist aus 2-Chlormethyl-5-chlorthiophen, 2-Chlormethyl-5-bromthiophen, Benzylchlorid, Methylchloracetat, Ethylchloracetat, α-Chlorphenylethan, α-Bromphenylethan, Chloracetonitril, Chloracetophenon, Methyl jodid, 1-Bromoctan, 1-Chloroctan, p-Chlorbenzylchlorid, Chlormethylthiophen, Chlormethylnaphthalin und Chlormethylfuran.

22. Verfahren nach einem der Ansprüche 1 bis 21, dadurch gekennzeichnet, daß die carboxylierte aromatische Verbindung der Formel (I) ein bicarboxylierte Verbindung ist, wenn im Ausgangshalogenid (II) eine $—CH_2X$ Gruppe anwesend ist, in welcher X aus Cl, Br und J ausgewählt ist.

23. Verfahren nach einem der Ansprüche 1 bis 22, dadurch gekennzeichnet, daß man in Anwesenheit eines hydro-alkoholischen Lösungsmittelmediums in wesentlichen die Alkali- oder Erdalkalisalze der carboxylierten aromatischen Verbindungen der Formel (I) erhält.

24. Verfahren nach einem der Ansprüche 1 bis 22, dadurch gekennzeichnet, daß man in Anwesenheit eines alkoholischen Lösungsmittelmediums im wesentlichen die Alkylester der carboxylierten aromatischen Verbindungen der Formel (I) erhält.

25. Verfahren nach einem der Ansprüche 1 bis 24, in welchem die Reaktion unter praktisch atmosphärischem Druck durchgeführt wird.

**Revendications**

1. Procédé de préparation de composés aromatiques ou hétéroaromatiques carboxylés de formule (I):

$$Y—Ar—\underset{\underset{O}{\|}}{C}—OR \qquad (I)$$

dans laquelle:

Ar représente un noyau aromatique possédant un ou plusieurs cycles benzéniques éventuellement (poly)condensés, ou un noyau hétéroaromatique éventuellement condensé; et dans laquelle:

Y représente un atome d'hydrogène, un halogène choisi parmi Cl, Br et I, un groupe alkyle, alkoxyle ou trifluoroalkyle possédant jusqu'à 4 atomes de carbone, un groupe ester, un groupe aryloxyle, un groupe nitrile, un groupe amide, un groupe acétamide, un groupe amine, ou un groupe acyle R″—CO— dans lequel R″ représente un groupe alkyle ou aryle; ou Y représente un groupe $—CH_2COOR$ dans lequel R représente un atome d'hydrogène, un groupe alkyl possédant jusqu'à 4 atomes de carbone ou dans lequel R représente un métal alcalin ou alcalinoterreux, caractérisé en ce que l'on fait réagir un halogénure aromatique de formule (II):

$$Y'—Ar—X \qquad (II)$$

dans laquelle:

Ar a la signification définie ci-dessus;

X représente un halogène choisi parmi Cl, Br et I; et

Y′ représente $—CH_2X$ ou Y dans lequel X et Y ont les significations définies ci-dessus, avec du

monoxyde de carbone dans un solvant alcoolique ou hydro-alcoolique et avec un dérivé accepteur d'acidité en présence d'un système catalytique comprenant:

(a) un sel de cobalt hydrocarbonyl ou un de ses précurseurs; et, si nécessaire,

(b) un halogénure de formule (III):

$$R'—X \qquad (III)$$

dans laquelle R' représente un groupe alkyle possédant jusqu'à 10 atomes de carbone, un groupe benzyle ou un groupe choisi parmi

$$—CH_2COOR, \quad —CH_2CN, \quad —CH_2—C(O)—Ar, \quad —CH_2—C(O)—CH_3,$$

$$—CH(CH_3)—Ar \quad et \quad —CH_2—Ar—Y$$

dans lequel Ar et R ont les significations définies ci-dessus, la présence de cet halogénure de formule (III) étant inutile lorsque l'halogénure aromatique de formule (II) qui réagit appartient à la classe des halogénure de formule (III) et a une température d'environ 20 à 80°.

2. Procédé selon la revendication 1, caractérisé en ce que le solvant est choisi parmi les alcools aliphatiques possédant jusqu'à 4 atomes de carbone, éventuellement en mélange avec de l'eau.

3. Procédé selon la revendication 2, caractérisé en ce que le solvant est l'alcool méthylique ou l'alcool éthylique.

4. Procédé selon l'une quelconques des revendications 1 à 3, caractérisé en ce que le dérivé accepteur d'acidité est une substance minérale ou organique basique.

5. Procédé selon la revendication 4, caractérisé en ce que la substance basique est choisie parmi les carbonates, oxydes et hydroxdes de métaux alcalins et alcalino-terreux et parmi les amines tertiaires.

6. Procédé selon la revendication 5, caractérisé en ce que la substance basique est choisi parmi les carbonates, oxydes et hydroxdes de Na, K, Ca et Mg et parmi la triéthylamine, la tétraméthylguanidine at leurs mélanges.

7. Procédé selon l'une quelconque des revendications 1 à 6, caractérisé en ce que le composant (a) du système catalytique est un sel de cobalt hydrocarbonyle de formule (IV).

$$Me^{n+}[Co(CO)_4]_n \qquad (IV)$$

dans laquelle Me représente un métal de valence n ou un précurseur d'un tel sel.

8. Procédé selon la revendication 7, caractérisé en ce que le métal Me est choisi parmi Na, K, Li, Co, Mn et Fe.

9. Procédé selon la revendication 7 ou 8, caractérisé en ce que le sel de cobalt hydrocarbonyle (a) est choisi parmi les sels de sodium, de cobalt, de fer et de manganèse.

10. Procédé selon l'une quelconque des revendications 7 à 9, caractérisé en ce que le sel (a) de cobalt hydrocarbonyle est préparé à partir d'un sel de cobalt, d'un alliage Fe-Mn en poudre contenant environ 80% de manganèse et à partir d'un promoteur sulfuré, dans l'alcool méthylique ou l'alcool éthylique, par réaction avec CO à une pression de 1 à 20 atmosphères et une température de 10 à 80°C.

11. Procédé selon la revendication 10, caractérisé en ce que ce sel de cobalt est un chlorure, un bromure ou un sulfate.

12. Procédé selon la revendication 10 ou 11, caractérisé en ce que ce promoteur sulfuré est un sulfure ou un thiosulfate alcalin.

13. Procédé selon l'une quelconque des revendications 10 à 12, caractérisé en ce que cette température de réaction est comprise entre 25 et 35°C.

14. Procédé selon l'une quelconque des revendications 7 à 9, caractérisé en ce que le sel alcalin de cobalt hydrocarbonyl (a) est préparé in situ à partir de $Co_2(CO)_8$ dans le milieu alcalin de la réaction.

15. Procédé selon l'une quelconque des revendications 1 à 14, caractérisé en ce que le rapport molaire entre l'halogénure aromatique de départ (II) et le composant (a) du système catalytique est compris entre 5/1 et 150/1.

16. Procédé selon la revendication 15, caractérisé en ce que le rapport molaire entre l'halogénure aromatique de départ (II) et le composant (a) du système catalytique est comprise entre 10/1 et 100/1.

17. Procédé selon l'une quelconque des revendications 1 à 16, caractérisé en ce que le rapport molaire entre l'halogénure aromatique de départ (II) et le composant (b) du système catalytique est compris entre 1/1 et 50/1.

18. Procédé selon la revendication 17, caractérisé en ce que le rapport molaire de l'halogénure aromatique de départ (II) et le composant (b) du système catalytique est compris entre 10/1 et 30/1.

19. Procédé selon l'une quelconque des revendications 1 à 18, caractérisé en ce que le dérivé accepteur d'acidité est présent en quantité qui est sensiblement stoechiométrique par rapport à l'équilibre de la réaction et peut atteindre un excès d'environ 50% en poids par rapport à cette quantité.

20. Procédé selon l'une quelconque des revendications 1 à 19, caractérisé en ce que l'halogénure aromatique de départ est choisi par les dérivés suivants: 2-chloronaphtaline, 2-chlorothiophène,

2-chloromethyl-5-chlorothiophène, 2-chlorométhyl-5-bromothiophène, bromobenzène, 4-chlorobenzonitrile, 1-chloronaphtaline, 4-bromotoluène, 4-bromoanisol, p-dibromobenzène, 4-chlorobromobenzène, méthyl-4-bromobenzoate, 2-bromothiophène, 2-bromoacétoanilide, 4-chlorobenzotrifluorure, 2-bromoaniline, 3-bromopyridine, p-bromobenzylchlorure, et 3-bromofuranne.

21. Procédé selon l'une quelconque des revendications 1 à 20, caractérisé en ce que le composant (b) du système catalytique est choisi parmi les dérivés suivants: 2-chlorométhyl-5-chlorothiophène, 2-chlorométhyl-5-bromothiophène, chlorure de benzyle, chloroacétate de méthyle, chloroacétate d'éthyle, α-chlorophényléthane, α-bromophényléthane, chloroacétonitrile, chloroacétophénone, iodure de méthyle, l-bromooctane, l-chlorooctane, chlorure de p-chlorobenzyle, chlorométhylthiophène, chlorométhylnaphtalène et chlorométhylfuranne.

22. Procédé selon l'une quelconque des revendications 1 à 21, caractérisé en ce que le dérivé aromatique carboxylé de formule (I) est un composé bicarboxylé lorsque dans l'halogénure (II) de départ existe un groupe $-CH_2X$ dans lequel X est choisi parmi Cl, Br et I.

23. Procédé selon l'une quelconque des revendications 1 à 22, caractérisé en ce que, en présence d'un milieu solvant hydro-alcoolique, on obtient pratiquement que des sels alcalins et alcalino-terreux des dérivés aromatiques carboxylés de formule (I).

24. Procédé selon l'une quelconque des revendications 1 à 22, caractérisé en ce que, en présence d'un milieu solvant alcoolique, on obtient pratiquement que des esters d'alkyles des dérivés aromatiques carboxylés de formule (I).

25. Procédé selon l'une quelconque des revendications 1 à 24, dans lequel cette réaction est effectuée à la pression sensiblement atmosphérique.